# EUROPEAN PATENT SPECIFICATION

(11) **EP 2 945 586 B1**
(45) Date of publication and mention of the grant of the patent: **15.07.2020**
(21) Application number: 14703917.6
(22) Date of filing: 15.01.2014
(51) Int. Cl.: A61F 13/08, A41B 11/04

(54) **SUPPORT STOCKING WITH HOOKING PROVISIONS**
KOMPRESSIONSSTRUMPF MIT HAKENBOHRUNGEN
BAS DE CONTENTION POURVU DE MOYENS D'ACCROCHAGE

(30) Priority: 15.01.2013 NL 2010130
(43) Date of publication of application: 25.11.2015
(73) Proprietor: Van Der Brugge, Frans, 4818 GS Breda (NL)
(72) Inventor: Van Der Brugge, Frans, 4818 GS Breda (NL)
(74) Representative: Hylarides, Paul Jacques
(86) International application number: PCT/NL2014/050018
(87) International publication number: WO 2014/112872

(56) References cited:
- WO-A1-2011/025396
- DE-U1- 29 816 222
- FR-A1- 2 587 617
- FR-A1- 2 956 314
- GB-A- 2 111 833
- GB-A- 191 122 391
- JP-A- 2011 017 097

## Description

The present invention relates to a support stocking configured for therapeutic compression and for exerting a pressure of at least 10mm Hg on the ankle of the user during use, wherein the support stocking is made of elastic material.

A support stocking, also referred to in the field as a therapeutic elastic stocking, is a stocking intended to exert extra pressure on a leg in that the stocking fits relatively tightly round the skin of the leg. This effect of the support stocking is also referred to as therapeutic compression. Drainage of fluid through the blood vessels is improved as a result of the compression, and accumulation of fluid (oedema) in the leg is prevented.

In the context of this description the term 'support stocking' comprises all known types of support stocking, including a sock (short) or a stocking (long), wherein the stocking can reach to the knee or to the groin.

The compression produced by support stockings is divided into different classes and is expressed in amount of pressure produced at the ankle. This achievable pressure lies in the range of 10 mm Hg (light) to 46 mm Hg (strong), and optionally even higher.

In determined embodiments the support stockings are embodied such that they can produce sufficient pressure for the CCL (Compression Class) relevant to the support stocking, for instance compression class CCLA (light 10-14 mmHg), CCL1 (mild 15-21 mmHg), CCL2 (normal 23-32 mmHg), CCL3 (strong 34-46 mmHg) or compression class CCL4 (extra-strong 49 > mmHg).

It is precisely because of the compressing property of a support stocking that it is difficult to pull on properly. The support stockings from the higher compression classes are particularly difficult to pull on. Pulling on requires considerable hand and arm strength and elderly people in particular often require assistance here from for instance a member of the household or a nurse. Aids such as rubber gloves can optionally be used during pulling on in order to improve the grip on the support stocking.

All in all it requires a relatively large amount of effort and time to pull on a support stocking properly, even when the above stated aids are used. The aids used here moreover have at best only a partially favourable effect.

Known from WO2011/025396 is a support stocking provided with a hooking provision in the form of a loop. The support stocking also has an opening which can be opened and closed with a zip. The support stocking has a foot portion. The heel is left free.

A stocking provided with a number of openings is known per se from FR 2 587 617 A1. The stocking is however a sports stocking and so is not of a type suitable for producing the above stated amount of pressure on the leg of the user. This known stocking is thus much easier to put on and the above stated drawback that pulling it on requires much strength does not occur in this known stocking.

It is an object of the invention to provide a support stocking which requires less effort and time to pull on. It is a further object of the invention to provide a support stocking for therapeutic compression (suitable for producing a pressure at the position of the ankle of at least 10 mm Hg) which can be pulled on easily by the user him/herself with one or two hands without appreciable risk of damage to the support stocking occurring.

According to an aspect of the invention, at least one of these objectives is achieved in a support stocking as defined in the appended claim 1. The support stocking according to this aspect of the invention can be put on easily and quickly by the user him/herself. The radial orientation allows the force exerted on the slit while the support stocking is being pulled on to be distributed over the length of the slit and not directly on an end point of the slit. This reduces the risk of damage to the support stocking, for instance tearing of the stocking at the position of the hooking provision.

A stocking provided with a number of openings is known per se from FR 2 587 617 A1. The stocking is however a sports stocking and so is not of a type suitable for producing the above stated amount of pressure on the leg of the user. This known stocking is thus much easier to put on and the above stated drawback that pulling it on requires much strength and that the pulling on entails a risk of tearing does not occur in this known stocking.

The support stocking is of a type suitable for therapeutic compression. According to an aspect of the invention, this is understood to mean a support stocking which can exert a pressure on the ankle of the user of at least 10 mm Hg.

In a further embodiment the slit has a form and dimensions such that one finger, or preferably two or more fingers at the same time, can be partially or wholly inserted therein. Pulling on will certainly be easy, and the risk of tearing particularly small, when two or more fingers can simultaneously exert force on the stocking.

The support stocking has one or more hooking provisions in the form of one or more openings through the material close to the upper end of the support stocking, wherein the opening comprises a reinforcing structure. The hooking provision is configured for manual pulling on of the support stocking. The hooking provisions are embodied such that one or more fingers can be hooked therein, so that the support stocking can be easily held fast at the upper end while the support stocking is being pulled on and pulled up. A person is in fact no longer dependent here on a good grip of the fingers on the material of the support stocking. The user thus effectively need not produce any squeezing force in order to properly hold the material fast between the fingers. The reinforcing structure is provided close to the edge of the opening, in an embodiment all around the whole opening. The reinforcing structure has the advantage that the force with which the opening is pulled during pulling on does not result in undesired tearing of the support stocking from the opening. The reinforcing structure is moreover able to distribute the force with which the opening is pulled over the material of the support stocking and so transmit this force over the greatest possible surface area.

The opening can for instance be embodied as a hole, wherein the edge is reinforced with stitching threads all around the hole. An additional reinforcing piece can moreover be stitched onto this edge if desired, preferably on the outer side of the support stocking. The reinforcing piece can for instance be of elastane.

In an embodiment the opening is arranged close to the upper end such that a reinforcing structure arranged close to the upper end functions as reinforcing structure of the upper end as well as reinforcing structure of the opening. A dual function is hereby obtained by arranging a single reinforcing structure.

The support stocking according to the invention is preferably pulled over at least the lower leg (calf).

The support stocking preferably comprises two hooking provisions so that using the left hand and the right hand a pulling force can be exerted on the upper end of the support stocking from two different positions. With particular preference the two hooking provisions are provided here on the left and right-hand side of the stocking.

In addition to the above stated embodiment of the hooking provision, the upper end of the support stocking can be provided with stitched-on loops of stiff material with relatively little elasticity. The loops are embodied such that one or more fingers can be hooked therein.

The reinforcing structure is preferably constructed from ribbon-like reinforcing pieces of stiff material with relatively little elasticity. Suitable material is for instance so-called twill tape which can be manufactured from cotton or synthetic fibre. The reinforcing pieces are preferably stitched onto the outer side of the stocking in the form of a reinforced structure. A V-shaped structure is recommended here, wherein the opening lies between the two legs of the V and the tip of the V points downward. Because the reinforcing structure is stitched to the outer side of the stocking, it does not interfere with the direct contact between the elastic material of the stocking and the lower leg.

The support stocking can be manufactured from machine-knit yarns. Yarns with a relatively high elasticity are used in order to produce sufficient compression. Examples of suitable materials comprise elastomers, such as elastane, as elastic component. These materials are often combined with cotton and/or polyamide fibres in order to provide a suitable yarn for a support stocking.

The high elasticity of the support stocking makes it possible for it to be given a relatively narrow form; i.e. the internal space in a support stocking when it is not being worn is considerably smaller than the girth of the leg inserted into the support stocking. This difference is many times greater than in normal stockings, which produce no more than a slight compression.

According to a favourable variant of the support stocking according to the invention, the opening takes the form of a slit, and the longitudinal direction of the slit preferably has a radial orientation on the support stocking.

The form of a slit allows the opening to close for the greater part once the stocking has been pulled on. This slit is also referred to here as a self-closing slit. This means that, when no force is being exerted on the slit by the fingers of the user, it tends to close itself, this not only having a positive effect on the appearance of the support stocking but also preventing the skin of the user tending to bulge outward at the position of the slits. The slit can for instance take a form similar to a buttonhole in clothing. The length of the slit is relatively great here, i.e. in the range of 2 cm to 5 cm, preferably about 4 cm. It is noted for the sake of completeness that the radial orientation on the support stocking is to be understood in the context of a support stocking with a tubular form having an approximately cylindrical shape.

It is noted in a general sense that the inner diameter of the support stocking according to the invention narrows to some extent in a manner corresponding to the narrowing of the girth of the lower leg.

In the support stocking according to the invention the hooking provision is more preferably present some distance from the upper end of the support stocking, wherein this distance lies in the order of magnitude of several centimetres.

This distance preferably lies in the range of 1 cm to 5 cm, and with particular preference is about 3 cm. A good distribution of forces over the support stocking is thus achieved when it is being pulled on, while the integrity of the support stocking is sufficiently protected against tearing.

The upper end of the support stocking can otherwise be embodied as a hem with a lower compression value than the main material of the support stocking. In this case it is recommended that the hooking provision is present at the position of the underside of this hem. The hooking provision thus provides a direct grip on the main material which produces the therapeutic compression.

In an embodiment self-securing means are arranged close to the upper end of the support stocking on an inner side of the support stocking. These can for instance be silicones arranged on an inner side of the support stocking. The support stocking is hereby better held in place around the leg.

The support stocking according to the invention is preferably of a type which produces therapeutic compression for the lower leg over the area between the knee and the foot, wherein the support stocking does not comprise a foot portion and thus has an open outer end on the upper side and underside.

Without foot portion the support stocking is easier to pull on because on the one hand the foot portion is difficult to position around the foot and on the other the support stocking as a whole has a larger inner surface area, whereby a greater frictional resistance occurs when it is pulled on.

It has moreover been tested, and established, that the therapeutic effect of the support stocking is obtained particularly by compression on the lower leg between knee and foot: this is because this area is readily compressible as the lower leg comprises a relatively large amount of soft parts such as fatty tissue, connective tissue and muscular tissue. The foot on the other hand is made up largely of bones, making therapeutic compression of the foot considerably less effective. All in all it has been found that a support stocking without foot portion can have a therapeutic effect comparable to that of a support stocking with foot portion of conventional type.

In a preferred embodiment of the support stocking without foot portion according to the invention the inner diameter of the support stocking is such that from a relatively narrow central zone with a small diameter it gradually increases toward the underside.

This design allows a slight decrease in the compression on the underside of the support stocking so that a support stocking which has been pulled on produces somewhat less compression at the position of the ankle joint. The transition zone between lower leg and foot is also referred to as the pretibial zone. Existing designs pinch relatively tightly in this zone, which results in problems when pulling the support stocking on and off. The same also applies as noted above for the foot as for the pretibial zone: due to the presence of a relatively large number of bone structures and relatively few soft parts the effect of therapeutic compression which can be obtained is rather low when compared to the lower leg. With a gradual widening of the underside of the support stocking without foot portion a suitable compromise is thus achieved between sufficient compression by the support stocking as a whole and simultaneously preventing the support stocking pinching uncomfortably at the position of the pretibial zone.

According to a variant of the support stocking without foot portion according to the invention, the edge of the underside of the support stocking is covered on the inner side with a band of elastic material.

A band of elastane is preferably used here. The band can for instance be stitched to the inner side and brings about a good fit on the ankle.

In a subsequent variant of the support stocking without foot portion according to the invention there is attached to the underside of the support stocking a heel loop in the form of a ribbon, the two outer ends of which are connected to respectively the left-hand side and right-hand side of the underside.

In an embodiment a lower end of the support stocking is provided with a heel portion. This heel portion is configured to partially enclose the heel of the wearer. The heel portion is formed close to the lower end of the support stocking, distally of the ankle part of the support stocking. The heel portion is configured to be arranged all around the heel of the patient. The heel portion comprises a part which is arranged under the sole of the foot of the patient. The heel portion thus encloses the rear part of the foot of the patient. This provides additional support. Partly because of the sole part, the heel portion also functions as positioning part, particularly limiting part, for the purpose of preventing the support stocking being pulled up too high. Such a heel portion is form-releasing, whereby arranging of the support stocking does not become more difficult. The support stocking can in particular be arranged first around the lower part of the leg, and the heel portion is only then placed around the heel.

The heel portion can be formed integrally from the elastic material of the support stocking. The heel portion is however preferably an attached piece of less elastic material with a convex form for the purpose of receiving the heel.

The lower end of the support stocking, provided in some embodiments with the heel portion, is preferably provided with a hem, in particular a hem band formed all around the whole lower end on the support stocking.

The ribbon is preferably of stiff material with relatively little elasticity. Suitable material is for instance twill tape which can be manufactured from cotton or synthetic fibre. The ribbon is preferably stitched onto the outer side of the support stocking. Because the ribbon is stitched onto the outer side of the stocking it does not interfere with the direct contact between the elastic material of the support stocking and the ankle.

The heel loop functions as an aid for setting and holding the support stocking at the correct height. Because the ribbon is of stiff material, the support stocking cannot ride upward during use, while the support stocking is automatically pulled up to a correct height when it is put on. This latter favourable effect is of greater relevance now that the support stocking according to the invention can be pulled up more easily because of the hooking provisions.

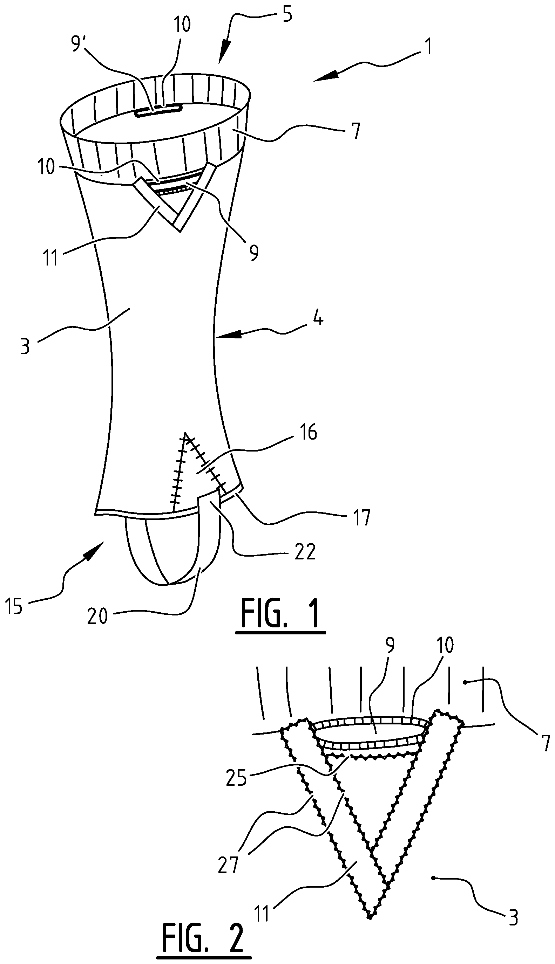

Examples of embodiments of the invention are given below on the basis of a description of the accompanying figures 1-3.

Figure 1 shows a support stocking constructed substantially from an elongate sleeve 3 of elastic material for therapeutic compression. The main material of sleeve 3 is a knitted yarn fabric and consists of 65% polyamide and 35% elastane. Sleeve 3 widens gradually from the narrowest part 4 in the direction of upper end 5 which is provided all around with a hem 7. Hem 7 is of a fabric which has a lower compression value than the main material of sleeve 3 of the support stocking. The height of the hem is about 3 cm.
Once support stocking 1 has been pulled on, hem 7 then covers the part of the lower leg of the user between the top of the calf and the knee.

Provided along the lower edge of hem 7 is a hooking provision in the form of a slit 9. A slit 9' is likewise provided on an opposite side. The periphery of the two slits is reinforced with stitching 10 in similar manner to a buttonhole. A V-shaped reinforcing structure 11 of cotton twill tape is provided on either side of slit 9. The length of slit 9 is about 4 cm and it has a radial orientation relative to sleeve 3 of the support stocking.

Sleeve 3 widens gradually from the narrowest part 4 in the direction of lower end 15. Once the support stocking has been pulled on, lower end 15 then covers the upper half of the ankle of the user, also referred to as the pretibial zone. Lower end 15 is widened by addition of an insert piece 16 made from the same material as the main material. Alternatively, the lower end can also be widened by adapting the method of knitting of sleeve 3 for this purpose. The edge of underside 15 of the support stocking is covered on the inner side with a band 17 of elastane.

Also attached to the underside of the support stocking is a heel loop 20 in the form of a ribbon of twill tape stitched at the two outer ends to respectively the left-hand side and right-hand side of underside 15 (wherein only one side can be seen in fig. 1).

Figure 2 shows a detail of the support stocking of figure 1 at the position of slit 9. The same components from figure 1 are designated with the same reference numerals. The V-shaped reinforcing structure 11 is fixed with stitching 27 to the main material of sleeve 3. Along the lower edge of slit 9 a reinforcing piece 25 of elastane is further stitched onto stitching 10 and onto the main material of sleeve 3.

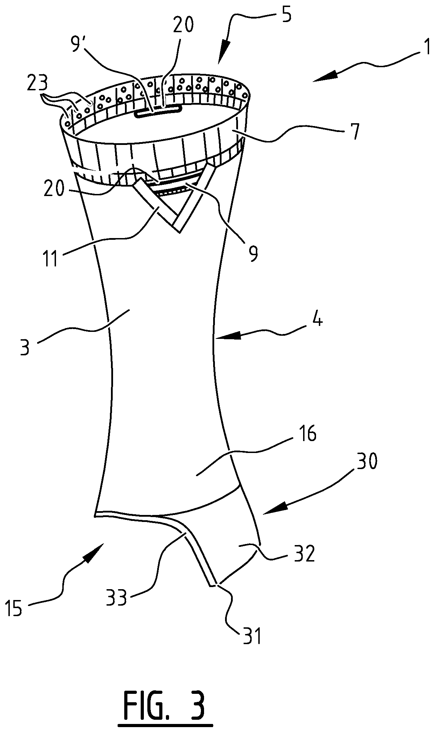

Figure 3 shows an alternative embodiment. The same components are designated with the same reference numerals and so as to avoid repetition reference is made to the previous embodiment for purposes of description.

Figure 3 shows a support stocking 1 with stitching 20 arranged around the whole of the support stocking close to upper end 5.

Stitching 20 connects elastic material 3 to hem 7. The inner side of hem 7 is provided with silicone beads 23 which together form self-securing means for holding the support stocking in place when it is being worn.

In the shown embodiment hem 7 also forms part of the periphery of slit-like opening 9. Other components of the periphery of opening 9 are formed by reinforcing structures 11.

A heel portion 30 is formed close to a lower end of support stocking 1. Heel portion 30 is formed in the shown embodiment by an intermediate part 32 which is connected to and placed between elastic material 3 and elastic material 31. Elastic material 31 is integrally connected to elastic material 3.

A hem 33 is formed on the lower end of elastic material 3 with elastic material part 31.

Heel portion 32 is formed such that the support stocking can be pulled on over the lower leg without the heel portion making this more difficult. The heel portion can be placed over the heel once the support stocking has been placed at the desired position around the lower leg.

## Claims

1. Support stocking (1) configured for therapeutic compression and for exerting a pressure of at least 10 mm Hg on the ankle of the user during use, wherein the support stocking is made of elastic material with an upper end comprising one or more hooking provisions (9) for manual pulling on of the support stocking, wherein the hooking provision comprises an opening through the material of the upper end of the support stocking, wherein the opening takes the form of a slit (9,9') having a longitudinal direction extending in radial orientation on the support stocking and a reinforcing structure (10) -arranged around a part of the slit (9,9'), wherein a slit has a form and dimensions such that two or more fingers at the same time, can be inserted therein.

2. Support stocking as claimed in claim 1, embodied such that they can produce sufficient pressure for compression class CCLA, CCL1, CCL2, CCL3, or CCL4, providing a pressure in the ranges 10-14 mmHg, 15-21 mmHg, 23-32 mmHg, 34-46 mmHg or more than 46 mmHg, respectively.

3. Support stocking as claimed in any of the foregoing claims, wherein the slit takes a self-closing form.

4. Support stocking as claimed in any of the foregoing claims, wherein two hooking provisions (9,9') are arranged on either of the support stocking close to the upper end.

5. Support stocking as claimed in any of the foregoing claims, wherein the hooking provision (9) is present at a distance of the order of magnitude of several centimetres from the upper end of the support stocking, wherein the distance lies in the range of 1 cm to 5 cm.

6. Support stocking as claimed in any of the foregoing claims, of a type which produces therapeutic compression for the lower leg only over the area between the knee and the foot, wherein the support stocking does not comprise a foot portion and thus has an open outer end on the upper side and underside.

7. Support stocking as claimed in claim 6, wherein the inner diameter of the support stocking is such that from a relatively narrow central zone with a small diameter it gradually increases toward the underside.

8. Support stocking as claimed in claim 6 or 7, wherein the edge (7) of the underside of the support stocking is covered on the inner side with a band (17) of elastic material.

9. Support stocking as claimed in any of the foregoing claims, wherein attached to the underside of the support stocking is a heel loop (20) in the form of a ribbon, the two outer ends of which are connected to respectively the left-hand side and right-hand side of the underside.

10. Support stocking as claimed in any of the foregoing claims, wherein the support stocking is provided with a heel portion formed close to a lower end of the support stocking and configured to receive the heel of a patient therein, and wherein the support stocking preferably further has no foot portion.

11. Support stocking as claimed in any of the foregoing claims, wherein the slit is arranged close to the upper end such that a reinforcing structure arranged close to the upper end functions as reinforcing structure of the upper end as well as reinforcing structure of the opening.

## Patentansprüche

1. Kompressionsstrumpf (1), der für eine therapeutische Kompression und zum Ausüben eines Drucks von wenigstens 10 mmHg auf den Knöchel des Benutzers während der Verwendung ausgestaltet ist, wobei der Kompressionsstrumpf aus einem elastischen Material mit einem oberen Ende, das eine oder mehrere Hakenbohrungen (9) aufweist, um den Kompressionsstrumpf manuell zu ziehen, hergestellt ist, wobei die Hakenbohrung eine Öffnung durch das Material des oberen Endes des Kompressionsstrumpfs aufweist, wobei die Öffnung die Form eines Schlitzes (9, 9') annimmt, der eine Längsrichtung, die in einer radialen Richtung an dem Kompressionsstrumpf verläuft, und eine Verstärkungsstruktur (10) hat, die um einen Teil des Schlitzes (9, 9') angeordnet ist, wobei ein Schlitz eine Form und Abmessungen dergestalt hat, dass zwei oder mehrere Finger gleichzeitig darin eingesetzt werden können.

2. Kompressionsstrumpf nach Anspruch 1, der so ausgeführt ist, dass er einen ausreichenden Druck für eine Komprimierungsklasse CCLA, CCL1, CCL2, CCL3 oder CCL4 jeweils in den Bereichen 10 - 14 mmHg, 15 - 21 mmHg, 23 - 32 mmHg, 34 - 46 mmHg oder über 46 mmHg, vorsehen kann.

3. Kompressionsstrumpf nach einem der vorhergehenden Ansprüche, wobei der Schlitz eine selbstschließende Form annimmt.

4. Kompressionsstrumpf nach einem der vorhergehenden Ansprüche, wobei die beiden Hakenbohrungen (9, 9') an jedem von dem Kompressionsstrumpf nahe dem oberen Ende angeordnet sind.

5. Kompressionsstrumpf nach einem der vorhergehenden Ansprüche, wobei die Hakenbohrung (9) an einem Abstand in der Größenordnung von einigen Zentimetern vom oberen Ende des Kompressionsstrumpfs vorhanden ist, wobei der Abstand im Bereich zwischen 1 bis 5 cm liegt.

6. Kompressionsstrumpf nach einem der vorhergehenden Ansprüche, wobei er derart ist, dass er eine therapeutische Komprimierung für das untere Bein nur über den Bereich zwischen dem Knie und dem Fuß erzeugt, wobei der Kompressionsstrumpf keinen Fußbereich aufweist und somit ein offenes äußeres Ende an der Oberseite und Unterseite hat.

7. Kompressionsstrumpf nach Anspruch 6, wobei der Innendurchmesser des Kompressionsstrumpfs dergestalt ist, dass er von einer relativ schmalen Mittelzone mit einem kleinen Durchmesser allmählich in Richtung der Unterseite größer wird.

8. Kompressionsstrumpf nach Anspruch 6 oder 7, wobei die Kante (7) der Unterseite des Kompressionsstrumpfs an der Innenseite mit einem Band (17) aus elastischem Material bedeckt ist.

9. Kompressionsstrumpf nach einem der vorhergehenden Ansprüche, wobei eine Fersenschleife (20) in Form eines Bandes an der Unterseite des Kompressionsstrumpfs angebracht ist, wobei die beiden äußeren Enden davon mit jeweils der linken Seite und der rechten Seite der Unterseite verbunden sind.

10. Kompressionsstrumpf nach einem der vorhergehenden Ansprüche, wobei der Kompressionsstrumpf mit einem Fersenbereich vorgesehen ist, der nahe einem unteren Ende des Kompressionsstrumpfs ausgebildet ist, und ausgestaltet ist, um die Ferse eines Patienten darin aufzunehmen, und wobei der Kompressionsstrumpf vorzugsweise keinen Fußbereich aufweist.

11. Kompressionsstrumpf nach einem der vorhergehenden Ansprüche, wobei der Schlitz nahe dem oberen Ende dergestalt angeordnet ist, dass eine Verstärkungsstruktur, die nahe dem oberen Ende angeordnet ist, als eine Verstärkungsstruktur des oberen Endes wie auch als eine Verstärkungsstruktur der Öffnung fungiert.

## Revendications

1. Bas de contention (1) configuré de manière à assurer une compression thérapeutique et à exercer une pression supérieure ou égale à 10 mmHg sur la cheville de l'utilisateur pendant l'utilisation, dans lequel le bas de contention est réalisé en un matériau élastique avec une extrémité supérieure comprenant un ou plusieurs agencements d'accrochage (9) afin d'assurer la tension manuelle du bas de contention, dans lequel l'agencement d'accrochage comprend une ouverture à travers le matériau de l'extrémité supérieure du bas de contention, dans lequel l'ouverture prend la forme d'une fente (9, 9') présentant une direction longitudinale s'étendant suivant une orientation radiale sur le bas de contention et une structure de renforcement (10) agencée autour d'une partie de la fente (9, 9'), dans lequel une fente présente une forme et des dimensions telles que deux ou plusieurs doigts peuvent être insérés simultanément à l'intérieur.

2. Bas de contention selon la revendication 1, mis en œuvre de telle sorte qu'il peut produire une pression suffisante pour les classes de compression CCLA, CCL1, CCL2, CCL3, ou CCL4, assurant respectivement une pression comprise dans les plages de 10 à 14 mmHg, de 15 à 21 mmHg, de 23 à 32 mmHg, de 34 à 46 mmHg ou supérieure à 46 mmHg.

3. Bas de contention selon l'une quelconque des revendications précédentes, dans lequel la fente présente une forme se refermant automatiquement.

4. Bas de contention selon l'une quelconque des revendications précédentes, dans lequel deux agencements d'accrochage (9, 9') sont agencés de part et d'autre du bas de contention, à proximité de l'extrémité supérieure.

5. Bas de contention selon l'une quelconque des revendications précédentes, dans lequel l'agencement d'accrochage (9) est présent à une distance de l'ordre de grandeur de plusieurs centimètres par rapport à l'extrémité supérieure du bas de contention, dans lequel la distance est comprise dans la plage de 1 cm à 5 cm.

6. Bas de contention selon l'une quelconque des revendications précédentes, d'un type qui produit une compression thérapeutique sur la jambe inférieure seulement sur la zone comprise entre le genou et le pied, dans lequel le bas de contention ne comporte pas de partie de pied et présente ainsi une extrémité externe ouverte du côté supérieur et du côté inférieur.

7. Bas de contention selon la revendication 6, dans lequel le diamètre interne du bas de contention est tel que, à partir d'une zone centrale relativement étroite présentant un faible diamètre, il augmente progressivement vers le côté inférieur.

8. Bas de contention selon la revendication 6 ou 7, dans lequel le rebord (7) du côté inférieur du bas de contention est recouvert sur le côté interne avec une bande (17) en un matériau élastique.

9. Bas de contention selon l'une quelconque des revendications précédentes, dans lequel une boucle de talon (20) sous la forme d'un ruban, dont les deux extrémités externes sont reliées respectivement sur le côté gauche et le côté droit du côté inférieur, est fixée sur le côté inférieur du bas de contention.

10. Bas de contention selon l'une quelconque des revendications précédentes, dans lequel le bas de contention comporte une partie de talon formée à proximité d'une extrémité inférieure du bas de contention et configurée de manière à recevoir, à l'intérieur, le talon d'un patient et dans lequel, plus préférablement, le bas de contention ne comporte pas de partie de pied.

11. Bas de contention selon l'une quelconque des revendications précédentes, dans lequel la fente est agencée à proximité de l'extrémité supérieure de telle sorte qu'une structure de renforcement agencée à proximité de l'extrémité supérieure fonctionne comme une structure de renforcement de l'extrémité supérieure de même que comme une structure de renforcement de l'ouverture.
